Europäisches Patentamt

European Patent Office

Office européen des brevets

(11) Publication number: **0 402 272 B1**

(12) **EUROPEAN PATENT SPECIFICATION**

(45) Date of publication of patent specification: **04.10.95**  (51) Int. Cl.6: **C12N 5/00**, C12M 3/00, C12M 1/12, A61M 1/36

(21) Application number: **90401581.5**

(22) Date of filing: **08.06.90**

(54) **Cell-culturing substrate, cell-culturing substrate unit bioreactor and extracorporeal circulation type therapeutic apparatus.**

(30) Priority: **09.06.89 JP 147336/89**
 **14.06.89 JP 151133/89**

(43) Date of publication of application:
 **12.12.90 Bulletin  90/50**

(45) Publication of the grant of the patent:
 **04.10.95 Bulletin  95/40**

(84) Designated Contracting States:
 **BE DE FR GB IT NL SE**

(56) References cited:
 **EP-A- 0 135 630      EP-A- 0 145 406**
 **EP-A- 0 392 010      WO-A-88/01279**
 **WO-A-88/09200       FR-A- 2 324 365**
 **US-A- 4 007 089**

(73) Proprietor: **TERUMO KABUSHIKI KAISHA**
 **No. 44-1, Hatagaya 2-chome,**
 **Shibuya-ku**
 **Tokyo 151 (JP)**

(72) Inventor: **Ohnishi, Makoto, C/o Terumo**
 **Kabushiki Kaisha**
 **1500 Inokuchi,**
 **Nakai-cho**
 **Ashigarakami-gun,**
 **Kanagawa-ken (JP)**
 Inventor: **Makino, Ayako, C/o Terumo**
 **Kabushiki Kaisha**
 **1500 Inokuchi,**
 **Nakai-cho**
 **Ashigarakami-gun,**
 **Kanagawa-ken (JP)**
 Inventor: **Shimura, Kenichi, C/o Terumo**
 **Kabushiki Kaisha**
 **1500 Inokuchi,**
 **Nakai-cho**
 **Ashigarakami-gun,**
 **Kanagawa-ken (JP)**

(74) Representative: **Gillard, Marie-Louise et al**
 **Cabinet Beau de Loménie**
 **158, rue de l'Université**
 **F-75340 Paris Cédex 07 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

Field of the Invention:

This invention relates to a cell-culturing substrate, a cell-culturing substrate unit, a bioreactor, and an extracorporeal circulation type therapeutic apparatus. More particularly, it relates to a cell-culturing substrate exhibiting satisfactory cell adhesion, spreading, and proliferation of hepatocytes which are difficult to culture, a unit of the cell-culturing substrate, a bioreactor for effecting a specific chemical reaction by the use of an immobilized microorganism as a catalyst, and an extracorporeal circulation type therapeutic apparatus for the treatment of diseases due to abnormal metabolism or failure of internal organs.

Description of the Prior Art:

The technique of culturing mammalian cells is an indispensable means for the production of physiologically active substances such as interferons, lymphokines, various growth hormones, and cell growth factors, materials of biotic origins, and therapeutic vaccines. In recent years, the high-density culture method for producing these substances with high efficiency in large amounts has been attracting attention. In the culture of mammalian cells, the high-density culture of culture-dependent cells required to adhere to an artificial or natural substrate for the purpose of ensuring survival and growth of the cells and production of a substance aimed at has as its primary requirement the fact that the surface of the substrate should excel in cell adhesion and in capability of aiding in the growth of the cells. For the cells to enjoy survival and retain their ability to produce the substance aimed at, how the environment favorable for the cells should be maintained constitutes an important question. It is, therefore, important to continue supply of nutrient and physiologically active substance and removal of wastes.

Concerning the cell adhesion to the cells it can be found, high molecular materials which have their surfaces treated by means of low-temperature plasma, sputtering, ultraviolet light, electron beam, and application of a monomolecular accumulated film [JP-B-58-32,589(1983), JP-A-63-198,976(1988), and JP-A-63-198,977(1988)] and materials treated with cell adhesion factors and cell growth factors such as collagen, fibronectin, vitronectin, and laminin [Trans. Am. Soc. Artif. Organs, Vol. 33, page 66 (1987) and Scholarly Reviews "Cell Adhesion to Biomaterials"].

As concerns the maintenance of the environment for the growth of high-density culture cells, the method which resorts to use of a porous membrane is believed to form an excellent measure. Specifically, by superposing flat porous membranes or bundling hollow fiber membranes, a large surface area can be secured within a limited inner volume. Then, the environment favorable for the growth of cells can be maintained by causing the supply of substances necessary for vital activity and the removal of extraneous substances to be forcibly continued through the micropores of the porous membranes by virtue of diffusion or pressure difference between membranes.

The term "bioreactor" refers to a reaction apparatus which, by the technique of bioreaction, carries out a chemical reaction such as degradation or synthesis of a substance in the presence of an enzyme as a catalyst. The enzymatic reaction, unlike the general chemical reaction, exhibits high specificity and possesses a characteristic that the reaction product is obtained with high efficiency under such mild conditions as normal room temperature and normal pressure. The technique of bioreactor, therefore, permits simplification of the reaction apparatus and reduction in the consumption of energy such as electric power. It is finding extensive utility in a rich variety of applications covering fermentation industry, textile industry, leather industry, foodstuff industry, and pharmaceutical industry, for example.

Incidentally, for commercial use of the enzymatic reaction, the practice of separating the enzyme from the substrate and the product and putting the separated enzyme to use again in a continuous operation of the reaction constitutes an essential requirement. Within the vital system, part of the enzyme stably exists in a granular form or in a state bound to a protein. When this enzyme is taken out of the vital system in the form of a solution, it is extremely unstable and is readily inactivated. Moreover, the separation of the enzyme and the reaction product from the reaction mixture is attained only with difficulty. Thus, the reclamation of the enzyme and the continuation of the reaction are extremely difficult. These difficulties have posed a serious hindrance to the dissemination of the bioreactor.

In the technique of bioreactor, therefore, easy separation of the enzyme from the substrate and the product has been realized by having the enzyme processed so as to be entrapped within a fixed space. The enzyme thus rendered separable is called an immobilized enzyme. The methods heretofore employed for the immobilization of the enzyme include the carrier binding method, the cross-linking method, and the embracing method, for example.

The technique of directly immobilizing an enzyme or microorganic cell bodies and using the immobilized microorganism as a catalyst for the bioreactor has been under study. This technique has an advantage that the microorganic cells are not required to be ruptured for extraction and isolation of the enzyme and the otherwise inevitable inactivation of the enzyme can be avoided. It has a further advantage that the composite enzyme system contained in the microorganic bodies and the cells can be utilized. Moreover, in the immobilized microorganism, since the cell membrane possesses improved permeability to substances, the reaction substrate and the product which normally fail to permeate the cell membrane are allowed to permeate the cell membrane of the immobilized microorganism to the extent of sufficiently enhancing the efficiency of the reaction.

As a way of remedying the symptoms arising from the impairment of detoxicating ability due to congenital enzyme deletion or acquired abnormal metabolism or from the internal accumulation of toxic substances, the therapeutic method resorting to use of enzyme has been arresting attention. Though this therapeutic method using the enzyme has the possibility of maturing into a curative means effective for numerous diseases, it has the following problems.

When an enzyme is to be internally used as a curative agent in the human body, it is required to have originated from a human being for the purpose of precluding serious secondary reactions accompanied by the antigen-antibody reaction. The enzymes which are currently available for use in the therapeutic method originate practically wholly from mammalians except for human beings or from microorganisms. For example, $\alpha$-1,4-glucosidase (medicine for glycogenosis type II due to abnormal intracorporeal accumulation of glycogen) originates from Aspergillus niger, $\alpha$-galactosidase (medicine for familial high-arginine blood arising from accumulation of arginine) originates in pig, and $\beta$-glucuronidase (medicine for mucosaccharogenesis VII) originates in the bovine liver.

Numerous culture substrates have been devised which enjoy improved cell adhesion to cells owing to modification of their surfaces by various surface-treating techniques represented by the treatment for impartation of hydrophilicity. Though most of these culture substrates are usable with such established cell lines as Hela (cancer of human cervix uteri), L (normal subcutaneous connective tissue in mouse), and BHK-21 (normal kidney of Syrian hamster), they find limited utility in liver parenchymous cells (hereinafter referred to as "hepatic cells") separated from the living body because they exhibit no sufficient cell adhesion or proliferation in their unmodified state. It is known that these substrates have their cell adhesion to hepatocytes and their expansibility improved by having their surfaces treated with collagen, ECM (intracellular matrix), cell adhesion factor, etc. By this method, however, the operation is complicated since the substrate is subjected to a coating treatment aseptically prior to use and the chance of ingression of contaminants is increased. Thus, this method is not a desirable approach. When the production of a useful substance by high-density culture is considered, this method is undesirable even from the economic point of view because the amounts of collagen and cell culturing factor required to be used so as to allow use of the membrane in a large amount are increased.

As concerns the hepatocytes, in the study of various specific functions owned by the liver, the primary-culture hepatocytes form a very powerful means in the place of mammalian experiments or established hepatocytes. While only part of the established cells retain their hepatically specific functions, most established cells have these functions totally lost or retained very slightly. Thus, the established cells have been unsatisfactory for the in vitro study of hepatically specific functions. In contrast, the primary-culture hepatocytes retain numerous hepatically specific functions as the in vivo liver and respond very favorably to numerous hormones. Thus a desire has been expressed for a substrate which excels in cell adhesion to hepatocytes and allows inexpensive and easy preparation of primary-culture hepatic cells.

In such serious hepatic diseases as violent hepatitis, since artificial substitution for high-order hepatic functions is attained only with difficulty, the hybrid type therapeutic artificial liver making combined use of hepatocytes and high molecular porous membranes has been proposed. In the circumstances, a desired has been expressed for a porous membrane which excels in the ability to allow culture and growth of hepatocytes and permit maintenance of hepatic functions.

In the case of the bioreactor and the extracorporeal circulation type therapeutic apparatus, the immobilization of a yeast or a microorganism entails numerous difficulties. When the carrier binding method or the cross-linking method is employed, for example, the cell membranes are heavily injured and the cell bodies suffer from leakage of enzyme. As a way of immobilizing a yeast or a microorganism, therefore, the embracing method which is capable of attaining the immobilization under relatively mild conditions is expected to serve as the most effective measure. In accordance with the carrier binding method, the binding effected in the form of covalent bond tends to impair the microorganic function and often impairs seriously the enzymatic activity, though it produces a large binding force. The simple ionic bond or physical adsorption tends to induce ready release of the enzyme because of weak binding force.

The technique of culture of mammalian cells is an indispensable means for the production of physiologically active substances such as interferons, lymphokine, various growth hormones, and cell growth factors, materials originating in living bodies, and therapeutic vaccines. In recent years, the method of high-density culture which produces such substances with high efficiency in large quantities has been attracting attention. In the culture of mammalian cells, the high-density culture of culture-dependent cells required to adhere to an artificial or natural substrate for the purpose of ensuring survival and growth of the cells and production of a substance aimed at has as its primary requirement the fact that the surface of the substrate should excel in cell adhesion to the cells and in capability of aiding in the growth of the cells. For the cells to enjoy survival and retain their ability to produce the substance aimed at, how the environment favorable for the cells should be maintained constitutes an important question. It is, therefore, important to continue supply of nutrient and physiologically active substance and removal of wastes.

Concerning the cell adhesion to the cells, it can be found high molecular materials which have their surfaces treated by means of low-temperature plasma, sputtering, ultraviolet light, electron beam, and application of a monomolecular laminate film [JP-B-58-32,589(1983), JP-A-63-198,976(1988) JP-A-63-198,977(1988)] and materials treated with cell adhesion factors and cell growth factors such as collagen, fibronectin, vitronectin, and laminin [Trans. Am. Soc. Artif. Organs, Vol. XXXIII, page 66 (1987) Scholarly Reviews "Cell Adhesion to Biomaterials"].

As concerns the maintenance of the environment for the growth of high-density culture cells, the method which resorts to use of a porous membrane is believed to form an excellent measure. Specifically, by superposing flat porous membranes or bundling hollow fiber membranes, a large surface area can be secured within a limited inner volume. Then, the environment favorable for the growth of cells can be maintained by causing the supply of substances necessary for vital activity and the removal of extraneous substances to be forcibly continued through the micropores of the porous membranes by virtue of diffusion or pressure difference between membranes. Concrete examples of this method are disclosed in JP-A-63-17,688(1988) (hollow fiber type) and JP-A-60-210,982(1985) (laminated type).

The membrane type cell culture apparatus is capable of culturing cells in a higher density than the cell culture method using a petri dish or a microcarrier. When this apparatus is used in culturing adhesion-dependent mammalian cells, however, the operation of the apparatus often encounters the following problems. Since the cells are cultured in a high density on a membrane, the produced substance is not necessarily recovered in a high yield and the functions of the cells are not maintained satisfactorily notwithstanding the culture of cells is effected by the use of a "membrane possessing a surface excellent in cell adhesion, expansibility, and proliferation". We, after a diligent study, have found that the poor results of the cell culture are caused when the propagated cells themselves and the extracellular substances (collagen, for example) synthesized by the cells occlude the micropores in the membrane to the extent of interfering the supply of necessary substances, the removal of extraneous substances, and the recovery of useful substances.

An object of this invention, therefore, is to provide a novel cell-culturing substrate, a cell-culturing substrate unit, a bioreactor, and an extracorporeal circulation type therapeutic apparatus.

Another object of this invention is to provide a cell-culturing substrate which excels in affinity for various cells and, even in the adhesion, expansion, and growth of hepatocytes heretofore regarded as difficult, manifests an outstanding performance without requiring any special collagen treatment and a unit using the substrate.

A further object of this invention is to provide a bioreactor capable of maintaining the activity of a microorganism for a long time and serving to enhance the efficiency of reaction.

Still another object of this invention is to provide an extracorporeal circulation type therapeutic apparatus enjoying high efficiency of the reaction, exhibiting highly satisfactory adaptability to blood, and ensuring ease of handling.

Yet another object of this invention is to provide a cell-culturing membrane possessing a surface structure excellent in cell adhesion and growth and exhibiting excellent performance with respect to supply of necessary substances, removal of extraneous substances, and recovery of useful substances and a cell-culturing apparatus using the membrane.

## SUMMARY OF THE INVENTION

The objects described above are accomplished by a microorganism- or cell-culturing substrate consisting of a high molecular substrate coated on at least one of its opposite surfaces by a synthetic high

EP 0 402 272 B1

molecular membrane layer made up of a (co)polymer of an $\alpha,\beta$-ethylenically unsaturated monomer possessing a nitrogen-containing heterocycle in the molecular unit thereof.

The present invention also discloses a cell-culturing substrate, wherein the high molecular membrane is a porous membrane. The present invention also discloses a cell-culturing substrate, wherein the monomer as the component of the synthetic high molecular membrane layer possesses in the molecular unit thereof a nitrogen-containing heterocycle in an amount such that the nitrogen atom/carbon atom ratio determined by the X-ray photoelectric spectrum is in the range of 0.01 to 0.3. The present invention also discloses a cell-culturing substrate, wherein the $\alpha,\beta$-ethylenically unsaturated monomer possessing in the molecular unit thereof the nitrogen-containing heterocycle is graft polymerized in a gaseous state on at least one of the opposite surfaces thereof. The present invention also discloses a cell-culturing substrate, wherein the high molecular substrate has as a main component theroof at least one member selected from the group consisting of polyolefins and polyolefins having part or whole of the hydrogen atoms thereof substituted with halogen atoms. The present invention also discloses a cell-culturing substrate, wherein the high molecular substrate has on at least one of the opposite surfaces thereof slender ridges as spaced with a substantially fixed interval.

The objects described above are also accomplished by a cell-culturing substrate unit, comprising at least two high molecular substrates of porous membrane combined as opposed to each other, a cell-culturing substrate having on at least one of the opposite surfaces of the high molecular substrate a synthetic high molecular membrane layer comprising an $\alpha,\beta$-ethylenically unsaturated monomer possessing a nitrogen-containing heterocycle in the molecular unit thereof, a seal formed along the matched peripheral parts of the high molecular substrates, and annular sealing members along the outer peripheries of supply mouths perforated in the substrates.

The present invention also discloses a cell-culturing substrate unit, wherein the monomer as the component of the synthetic high molecular membrane layer possesses in the molecular unit thereof a nitrogen-containing heterocycle in an amount such that the nitrogen atom/carbon atom ratio determined by the X-ray photoelectric spectrum is in the range of 0.01 to 0.3. The present invention also discloses a cell-culturing substrate unit,. wherein the $\alpha,\beta$-ethylenically unsaturated monomer possessing in the molecular unit thereof the nitrogen-containing heterocycle is graft polymerised in a gaseous state on at least one of the opposite surfaces thereof. The present invention also discloses a cell-culturing substrate unit, wherein the high molecular substrate has as a main component thereof at least one member selected from the group consisting of polyolefins and polyolefins having part or whole of the hydrogen atoms thereof substituted with halogen atoms. The present invention also discloses a cell-culturing substrate unit, wherein the high molecular substrate has on at least one of the opposite surfaces thereof slender ridges as spaced with a substantiallY fixed interval.

The objects described above are also accomplished by bioreactor comprising a first compartment and a second compartment for a reaction solution, a microorganism- or cell-culturing substrate having on at least one of the opposite surfaces of a high molecular substrate of porous membrane a synthetic high molecular membrane layer using as a component thereof an $\alpha,\beta$-ethylenically unsaturated monomer possessing a nitrogen-containing heterocycle in the molecular unit thereof, the microorganism- or cell-culturing substrate serving to partition the first and second compartments, and further comprising a reaction solution-exchanging mouth in the second compartment and adapted to effect exchange of the reaction solution for at least one member selected from the group consisting of microorganisms and cells, stowed in the first compartment.

The present invention also discloses a bioreactor, wherein the monomer as the component of the synthetic high molecular membrane layer possesses in the molecular unit thereof a nitrogen-containing heterocycle in an amount such that the nitrogen atom/carbon atom ratio determined by the X-ray photoelectric spectrum is in the range of 0.01 to 0.3. The present invention also discloses a bioreactor, wherein the high molecular substrate of porous membrane is in the form of hollow fibers and the outside of the microorganism- or cell-culturing substrate having the synthetic high molecular membrane layer formed on the surface of the hollow-fiber substrate constitutes itself the first compartment. The present invention also discloses a bioreactor, wherein the high molecular substrate of porous membrane is in the form of a flat membrane and the first compartment is formed of the interior of a cell-culturing substrate unit comprising at least two high molecular substrates of porous membrane combined as opposed to each other, a cell-culturing substrate having formed in at least one of the opposite surfaces of the high molecular substrate a synthetic high molecular membrane layer comprising as a component thereof an $\alpha,\beta$-ethylenically unsaturated monomer possessing a nitrogen-containing heterocycle in the molecular unit thereof, having a seal along the matched peripheral parts of the high molecular substrates, and annular sealing members along the outer peripheries of supply mouths perforated in the substrates.

5

The objects described above are also accomplished by an extracorporeal circulation type therapeutic apparatus, comprising a first compartment and a second compartment for a reaction solution, a microorganism- or cell-culturing substrate having on at least one of the opposite surfaces of a high molecular substrate of porous membrane a synthetic high molecular membrane layer having as a component thereof an $\alpha,\beta$-ethylenically unsaturated monomer possessing a nitrogen-containing heterocycle in the molecular unit thereof, the microorganism- or cell-culturing substrate serving to partition the first and second compartments, at least one member selected from the group consisting of microorganisms and cells is stowed in the first compartment, and an outlet and an inlet for perfusing blood to the second compartment. The present invention also discloses an extracorporeal circulation type therapeutic apparatus, wherein the high molecular substrate of porous membrane possesses through holes having an average pore diameter in the range of 5 Å to 10 $\mu$m. The present invention also discloses an extracorporeal circulation type therapeutic apparatus, wherein the monomer as the component of the synthetic high molecular membrane layer possesses in the molecular unit thereof a nitrogen-containing heterocycle in an amount such that the nitrogen atom/carbon atom ratio determined by the X-ray photoelectric spectrum is in the range of 0.01 to 0.3. The present invention also discloses an extracorporeal circulation type therapeutic apparatus, wherein the high molecular substrate of porous membrane is in the form of hollow fibers and the outside of the microorganism- or cell-culturing substrate having the synthetic high molecular membrane layer formed on the surface of the hollow-fiber substrate constitutes itself the first compartment. The present invention also discloses an extracorporeal circulation type therapeutic apparatus, wherein the high molecular substrate of porous membrane is in the form of a flat membrane and the first compartment is the interior of a cell-culturing substrate unit comprising at least two high molecular substrates of porous membrane combined as opposed to each other, a cell-culturing substrate having formed on at least one of the opposite surfaces of the high molecular substrate a synthetic high molecular membrane layer having as a component thereof an $\alpha,\beta$-ethylenically unsaturated monomer possessing a nitrogen-containing heterocycle in the molecular unit thereof, a seal along the matched peripheral parts of the high molecular substrates, and annular sealing members along the outer peripheries of supply mouths perforated in the substrates.

The present invention is constructed as described above. The cell-culturing substrate according with the present invention excels in cell adhesion, extensibility, and proliferation because the synthetic high molecular membrane having as a component thereof an $\alpha,\beta$-unsaturated ethylenically unsaturated monomer possessing a nitrogen-containing heterocycle in the molecular unit thereof is retained on the surface of the substrate. The exact reason for this excellent quality remains yet to be elucidated. By preparing various surfaces synthetically and evaluating them as to their affinity for cells, the inventors have demonstrated that, as observed in pyridine and other similar nitrogen-containing heterocycles, side chains exhibit weak cationic activity due to the secondary or tertiary nitrogen, assume a resonant energy (cyclic conjugate system) owing to delocalization of electron, and possess suitable hydrophobicity. It is believed that these side chains go to enhance cell adhesion and proliferation. When the nirtrogen in the nitrogen-containing heterocycle is quaternized, the state of adhesion of cells can be controlled by the intensity of hydrophobicity of the quaternizer (halogenide).

The cell-culturing substrate of the present invention has no possibility of being dissolved out in the culture solution or the blood or being separated by peeling because the synthetic high molecular membrane having as a component thereof an $\alpha,\beta$-ethylenically unsaturated monomer possessing a nitrogen-containing heterocycle in the molecular unit thereof is bound by surface graft polymerization through the medium of covalent bond to the high molecular substrate. When the monomer supplied in a gaseous form is polymerized by solid-gas phase graft polymerization, a graft chain having high affinity for surface cells can be introduced into the cell-culturing substrate possessing a delicate and complicate shape (made of a porous material or a material endowed with a jogging contour and consequently an increased surface area) without a sacrifice of the physical properties of the high molecular substrate. When the high molecular substrate uses as its main component a polyolefin or a polyolefin having part or whole of hydrogen atoms thereof halogenated, the cell-culturing substrate not only allows surface grafting to proceed easily but also excels in resistance to enzymatic degradation (resistance to biodegradation) and resistance to chemicals. Thus, it is rendered possible to produce a cell-culturing substrate which can be used for protracted culture without encountering any appreciable deterioration by aging due to exposure to the product of metabolism or the product of fermentation. Owing to the characteristics described above, the use of the cell-culturing substrate according with the present invention allows fabrication of a bioreactor of highly desirable behavior. This bioreactor can be utilized in a system for the production of various physiologically active substances. It further contributes to biological and medical researches and serves effectively as artificial liver and artificial pancreas of the hybrid type allowing coexistence of high molecular substance and cells. Moreover, the bioreactor of the present invention is also useful as an extracorporeal circulation type therapeutic apparatus.

The objects described above are further accomplished by a cell-culturing porous membrane characterized by possessing in at least the cell-culturing side membrane surface a region prohibiting adhesion of cells and a region allowing adhesion of cells. The objects are also accomplished by a cell-culturing apparatus using the aforementioned cell-culturing porous membranes. The present invention does not specifically discriminate the cell-culturing membrane on account of its shape. The cell-culturing membrane may be in the form of a flat membrane, a hollow fiber, or a tube, for example. The expression "cell-culturing side surface" as used herein refers to the surface which is destined to contact the cells.

It is clear from the description given above that the cell-culturing porous membrane of the present invention is provided on the surface thereof with a region inhibiting adhesion of cells and, therefore, has no possibility of sustaining damage to the function thereof or encountering hindrance to supply and recovery of necessary substances by having the pores in the membrane clogged with the cells adhering to the membrane or with the substance produced by the cells. The cell-culturing apparatus using the cell-culturing porous membrane of the present invention, therefore, allows culture of cells and recovery of useful substances to be carried out efficiently in high density for a long time and manifests its effect in the system for producing various physiologically active substances with cells, in the biological and medical researches, and as artificial liver and artificial pancrease of the hybrid type allowing coexistence of a high molecular substance and cells.

BRIEF DESCRIPTION OF THE INVENTION

Fig. 1 a to Fig. 1 d are magnified cross sections illustrating examples of a flat-membrane type high molecular substrate for use in a cell-culturing substrate of the present invention,
Fig. 2 is a plan view illustrating one example of a cell-culturing substrate unit of the present invention,
Fig. 3 is a magnified cross section taken through Fig. 2 along the line III-III,
Fig. 4 is a magnified cross section illustrating another embodiment of this invention similarly as in Fig. 3,
Fig. 5 is an exploded perspective view illustrating one example of a bioreactor of the present invention,
Fig. 6 is an exploded perspective view illustrating another example of the bioreactor,
Fig. 7 is a partially cutaway perspective view illustrating still another example, and
Fig. 8 is a schematic cross section illustrating yet another example.

EXPLANATION OF THE PREFERRED EMBODIMENT

The cell-culturing substrate of this invention has on at least one of the opposite surfaces of a high molecular substrate a synthetic high molecular membrane having as a component thereof an $\alpha,\beta$-ethylenically unsaturated monomer possessing a nitrogen-containing heterocycle in the molecular unit thereof.

The high molecular substrates which are usable herein include substrates made of such thermoplastic resins as regenerated cellulose, cellulose derivatives, polyethylene, polypropylene, polystyrene, polymethyl methacrylate, polycarbonate, polyurethane, polyvinyl chloride, polyvinyl bromide, polyethylene terephthalate, polyamide, polytetrafluoroethylene, polyvinylidene chloride, and polysulfones, and copolymers of the thermoplastic resins and blends thereof. A substrate using as a main component thereof a polyolefin excellent in resistance to enzymatic degradation and in resistance to chemicals or a polyolefin having part or whole of the hydrogen atoms thereof substituted with a halogen proves to be preferable. This invention does not particularly discriminate the high molecular substrate on account of its form. The forms which are allowable herein include petri dish, bottle, non-woven fabric, woven fabric, film, flask, tube, hollow fiber, bag, block, minute particles, and combinations thereof, for example. For use in protracted cell culture or in cell culture performed in a state approximating the in vivo state, porous substrates possessing minute pores facilitating metabolism and assuming the forms of flat membrane, hollow fiber, tube, non-woven fabric, etc. prove to be particularly preferable among other forms mentioned above. In consideration of such factors as recovery of useful substance, modulation of construction, retention of cell components, and velocity of mass transfer, the porous membrane in the form of hollow fiber or flat web is preferable. The pores in the membrane have an average diameter in the range of 0.02 to 2 $\mu$m, preferably 0.05 to 1.0 $\mu$m, the wall thickness of the membrane is in the range of 10 to 1,000 $\mu$m, preferably 10 to 300 $\mu$m, and the porosity of the membrane is in the range of 20 to 90%, preferably 40 to 80%.

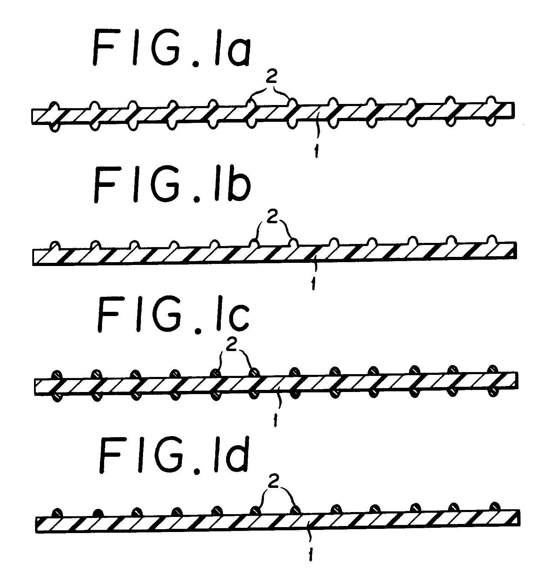

The flat membrane type high molecular substrate may be in a substantially perfectly flat shape. It may be provided on at least one surface of the opposite surfaces thereof with a plurality of minute projections 2 as illustrated in Fig. 1a to Fig. 1d. The flat membrane type substrate of the present invention may be provided with the plurality of minute projections 2 on only one of the opposite surfaces thereof as illustrated in Fig. 1 b and Fig. 1 d or on both opposite surfaces thereof as illustrated in Fig. 1 a and Fig. 1 c. The choice between the two patterns just mentioned depends on the position for setting the high molecular substrate or on the kind of use. The minute projections 2 may be in the shape of dots, lines, a lattice, etc. and are not particularly discriminated on account of shape. The height, H, of the minute projections 2 is preferable to be in the range of 20 to 1,000 $\mu$m, preferably 60 to 200 $\mu$m. The ratio of the area occupied by the minute projections 2 to the total surface area of the surface containing these minute projections 2 is preferable to be in the range of 0.5 to 50%, preferably 1.0 to 20%. If the height, H, of the minute projections 2 is less than 20 $\mu$m, the minute projections 2 fail to function sufficiently as a spacer or retain a stable thickness for the flow path. Conversely, if the height, H, of the minute projections 2 exceeds 1,000 $\mu$m, the minute projections 2 tend to sustain heavy deformation and involve an error and pose a problem in terms of priming volume. If the area occupied by the minute projections 2 is less than 0.5%, the high molecular substrate cannot be fully prevented from deformation. Conversely, if this area exceeds 50%, the surface area of the substrate available for permeation is decreased possibly to the extent of preventing the substrate from manifesting sufficient permeability.

In the flat membrane type high molecular substrate of the present invention, the minute projections 2 may be formed of the same material as the main body of the high molecular substrate 1 as illustrated in Fig. 1 a and Fig. 1 b or formed of a different material from the material of the main body of the high molecular substrate as illustrated in Fig. 1 c and Fig. 1 d. Preferably, the minute projections 2 are made of a material whose Young's modulus is in the range of $1.0 \times 10^6$ to $2.0 \times 10^{10}$ dynes/cm$^2$, preferably $1.0 \times 10^6$ to $1.0 \times 10^9$ dynes/cm$^2$. The reason for this particular range of Young's modulus is that when two or more such flat membrane type high molecular substrates are superposed as in a module, the flow path formed by the space intervening between the adjacent high molecular substrates 2 can be easily narrowed under an applied pressure and, on relief of the pressure, can be spontaneously restored so as to allow easy acquisition of the desired amount of permeation. Generally, in the laminated type of module described above, it is known that the shear rate is increased, the range of concentration distribution is decreased, and the permeation characteristic is consequently improved in proportion as the thickness of the flow path for the solution under treatment is decreased. Where the thickness of the flow path is varied with the magnitude of the applied pressure, the adjustment of the flow path to the desired thickness is obtained with difficulty if the minute projections 2 are formed of a material possessing a Young's modulus deviating from the range. Then, there arises the possibility that the module will fail to acquire the desired amount of permeation.

The flat membrane type high molecular substrate constructed as described above is produced as follows. Where the membrane is to be obtained by casting a fluid dope in the shape of a flat sheet and allowing the flat sheet of dope to solidify, the formation of a plurality of minute projections on at least one of the opposite surfaces of the permeable membrane is attained by allowing the corresponding surface of the flat sheet of dope to remain in contact with a mold surface possessing as many minute depressions as the minute projections while the flat sheet of dope is left solidifying. This method of production can be effectively employed then the permeable membrane body 1 and the minute projections 2 are formed severally of a material capable of forming a high molecular substrate and solidifiable from a fluid state such as a melt or a solution into a solid state through the process of solidification as by cooling, by extraction from a solvent, or by reversal of derivation, for example. The impartation of permeability to the membrane is not particularly discriminated on account of the kind of means to be employed. This impartation may be attained by extraction from solvent, removal of filler, mechanical elongation, etc. The mold possessing a surface provided with a plurality of minute depressions and fused in the aforementioned method of production is not specifically discriminated on account of its shape. The mold may be in the shape of a flat plate or a roller. The shape of the mold may be suitably selected, depending on the process of solidification of the fluid dope. The mold surface provided with the plurality of minute depressions is transferred to the surface of the permeable membrane when the sheet of fluid dope is solidified into a membrane and consequently enabled to give rise to the desired surface of minute projections on the produced permeable membrane. In conformity with the shape of the minute projections to be produced, therefore, the depth of the minute depressions is in the range of 20 to 1,000 $\mu$m, preferably 60 to 200 $\mu$m and the ratio of the area occupied by the minute depressions to the total surface area of the mold surface is in the range of 0.5 to 50%, preferably 1.0 to 20%.

The synthetic high molecular compound having as a component thereof an $\alpha,\beta$-ethylenically unsaturated monomer possessing a nitrogen-containing heterocycle in the molecular unit thereof may be a homopolymer of an $\alpha,\beta$-ethylenically unsaturated monomer containing a nitrogen-containing heterocycle or a copolymer of the monomer with other copolymerizable monomer. The $\alpha,\beta$-ethylenically unsaturated monomers containing a nitrogen-containing heterocycle which are usable in the present invention include 1-vinyl pyrazole, 3,5-dimethyl-1-vinyl pyrazole, 3-methyl-5-phenyl-1-vinyl pyrazole, 1-phenyl-4-vinyl pyrazole, 5-vinyl pyrazoline, 3-methyl-5-vinyl pyrazoline, 1-vinyl imidazole, 2-vinyl imidazole, 4-vinyl imidazole, 1-methyl-2-vinyl imidazole, 1-vinyl-2-methyl imidazole, 1-methyl-2-vinyl imidazole, 1-methyl-5-vinyl imidazole, 1-ethyl-5-vinyl imidazole, 2-ethyl-5-vinyl imidazole, 1-propyl-5-vinyl imidazole, 1-butyl-5-vinyl imidazole, 2-vinyl-2,4-dimethyl imidazole, 1-vinyl-2-imidazoline, 1-vinyl-2-methyl-2-imidazoline, 2-vinyl-3-methyl-2-imidazoline, 1-vinyl-2-imidazolinone, 3-vinyl-5,5-dimethyl hydantoin, 1-vinyl-2-pyrrolidone, 3-vinyl-2-pyrrolidone, 1-vinyl-3-methyl-2-pyrrolidone, 1-vinyl-4-methyl-2-pyrrolidone, 1-vinyl-5-methyl-2-pyrrolidone, 1-vinyl-3,3-dimethyl-2-pyrrolidone, 1-vinyl-5-phenyl-2-pyrrolidone, 1-vinyl-3-benzyl-2-pyrrolidone, 2-vinyl-2-oxazoline, 4-methyl-2-vinyl-2-oxazoline, 5-methyl-2-vinyl-2-oxazoline, 2-vinyl-2,3-dimethyl oxazoline, 5-methyl-N-vinyl-1,3-oxazolidin-2-on, N-vinyl-2,2,5,5-tetramethyl-4-oxazolidone, 3-methyl-4-isopropenyl isooxazole, 3-vinyl-1,3-oxazin-2-on, 3-vinyl-1,2,4-oxadiazole, 2-vinyl-1,2,4-triazole, 2-vinyl-1,2,3-triazole, 4-vinyl-1,2,3-triazole, 1-vinyl tetrazole, 2-vinyl tetrazole, 1-methyl-5-vinyl tetrazole, 2-methyl-5-vinyl tetrazole, 1-vinyl indole, 3-methyl-1-vinyl indole, N-vinyl benzotriazole, 2-vinylbenzoimidazole, N-vinyl benzoimidazole, N-vinyl-2-methyl benzoimidazole, 2-vinyl imidazole, 9-vinyl carbazole, 2-vinyl pyridine, 3-vinyl pyridine, 4-vinyl pyridine, 2-vinyl-3-methyl pyridine, 2-vinyl-4-methyl pyridine, 5-vinyl-2-methyl pyridine, 4-vinyl-3,5-dimethyl pyridine, 3-vinyl piperidine, 1-methyl-2-vinyl piperidine, 4-vinyl pyrimidine, 2-N,N-dimethylamino-4-vinyl pyrimidine, 2-vinyl pyrazine, 2-vinyl-3,4,5,6-tetrahydropyrimidine, 1-vinyl-tetrahydropyrimidine, 5,6-dihydro-2-vinyl-4H-1,3-oxazine, 1-vinyl-2-piperidone, 3-vinyl-1,3-oxazin-2-on, 4-vinyl-3-morpholinone, N-vinyl-N,N-trimethylene urea, 3-(2-vinyl)-6-methyl-4,5-dihydropyrrolidinone, 2,4-dimethyl-6-vinyl-1,3,5-triazine, 2,4-diamino-6-vinyl-1,3,5-triazine, 1-vinyl-3,5-dibutyl isocyanurate, 1-vinyl-2-ethoxy-4-pyrimidine, 1-vinyl uracil, 1-vinyl-3-methyl uracil, 6-methyl-1-vinyl uracil, 1-vinyl thymine, 2-vinyl quinoline, 4-methyl-4-vinyl quinoline, 9-vinyl acridine, 9-vinyl adenine, 6-acetyl-9-vinyl adenine, 9-vinyl hypoxanthine, 9-vinyl guanine, 5-methyl-2-vinyl imino divenzyl, N-vinyl phthalimide, and N-vinyl succinimide, for example.

The nitrogen-containing heterocycle in any of these monomers may be quaternized with a suitable halogenide. It may be otherwise in the form of a salt.

The monomers copolymerizable with the aforementioned $\alpha,\beta$-ethylenically unsaturated monomers containing a nitrogen-containing heterocycle include acrylic acid, methyl acrylate, ethyl acrylate, isopropyl acrylate, butyl acrylates, methacrylic acid, methyl methacrylate, ethyl methacrylate, isopropyl methacrylate, butyl methacrylates, 2-hydroxyethyl acrylate, 2-hydroxymethacrylate, acrylamide, methacrylamide, acrylonitrile, methacrylonitrile, vinyl acetate, and styrene, for example.

The cell-culturing substrate of the present invention is preferred to have retained at least on the surface of the high molecular substrate a synthetic high molecular compound having as a component thereof a monomer possessing a nitrogen-containing heterocycle in the molecular unit thereof in such an amount that the nitrogen atom/carbon atom ratio in the cell-culturing surface of the high molecular substrate determined by the X-ray photoelectric spectrum is in the range of 0.01 to 0.3, preferably 0.02 to 0.2. The expression "nitrogen atom/carbon atom ratio in the cell-culturing surface determined by the X-ray photoelectric spectrum" as used herein refers to the value of $N_{1S}/C_{1S}$ determined by the measurement with a spectrometer (produced by Japan Electron Optics Laboratory Co., Ltd. and marketed under product code of "JPS90-SX") using $\theta = 90°$. The nitrogen atom/carbon atom ratio in the membrane surface forms the index of the amount of the nitrogen-containing heterocycle present in the membrane surface. If this value is less than 0.01, no sufficient effect of the presence of the heterocycle is obtained. If this value exceeds 0.3, the presence of the heterocycle proves to be excessive. When the nitrogen atom/carbon atom ratio is not more than 0.3, the membrane surface tolerates the presence of nitrogen atom besides the nitrogen atoms originating in the nitrogen-containing heterocycle.

The retention in the substrate surface of the synthetic high molecular compound having as a component thereof an $\alpha,\beta$-ethylenically unsaturated monomer containing a nitrogen-containing heterocycle is not particularly discriminated on account of the method to be adopted therefor but may be attained by the method of coating the surface with a solution of the high molecular compound synthesized in advance, the method of insolubilizing the high molecular compound solution by cross-linking, the method of graft polymerization of the monomer mentioned above, or the method of plasma polymerization. The retention which is attained by causing the aforementioned monomer to be supplied in the gaseous state to the high molecular substrate possessing in at least part of the surface thereof a polymerization initiating point capable of graft polymerization and then subjected to surface graft polymerization proves to be desirable. The expression "graft polymerization initiating point" as used herein refers to the state of a monomer possessing a double bond in the molecular unit thereof and consequently exhibiting an ability to induce radical polymerization. To meet this description, the monomer is required to be capable of forming a high molecular radical on the membrane surface. The agents which are available for the formation of the high molecular radical include electron beam, gamma ray, ultraviolet light, plasma, ozone, and radical-forming agent (dehydrogenating agent), for example.

The method using electron beam or plasma proves to be preferable because it enables the monomer to be supplied in the gaseous state, allows the surface graft polymerization to proceed in the form of solid-gas phase polymerization, and permits the cell-culturing substrate to be produced in a dry process.

The thickness of the synthetic high molecular membrane formed as described above from the $\alpha,\beta$-ethylenically unsaturated monomer containing a nitrogen-containing heterocycle is not more than 100 $\mu$m, preferably not more than 1 $\mu$m.

The cell-culturing substrate obtained as described above is suitably used, depending on the form of the high molecular substrate to be used therein. Specifically, (1) the film type substrate in the form of a laminate or a roll or in its unmodified form, (2) the tube type substrate, (3) the filament type substrate, and (4) the woven or non-woven fabric type substrate severally in the form of a laminate, a roll, or a pack of divided pieces, (5) the bead type substrate in the form of floating particles or a column packed with particles, and (6) the block type substrate in the form of a collection or pack of small shaped pieces are each caused to have cells deposited on the surface of synthetic high molecular membrane and then immersed in a culture solution to induce culture of the cells. Besides, (7) the buckle type substrate, (8) the bottle type substrate, and (9) the dish type substrate each in the form having the synthetic high molecular membrane layer superposed on the inner surface side are each caused to have cells deposited on the membrane layer and then immersed in a culture solution to induce culture of the cells. Otherwise, (10) in a container having the synthetic high molecular membrane layer formed on the inner surface thereof, the cell-culturing substrate of any of the types (1) to (6) mentioned above is placed and used for culture of the cells.

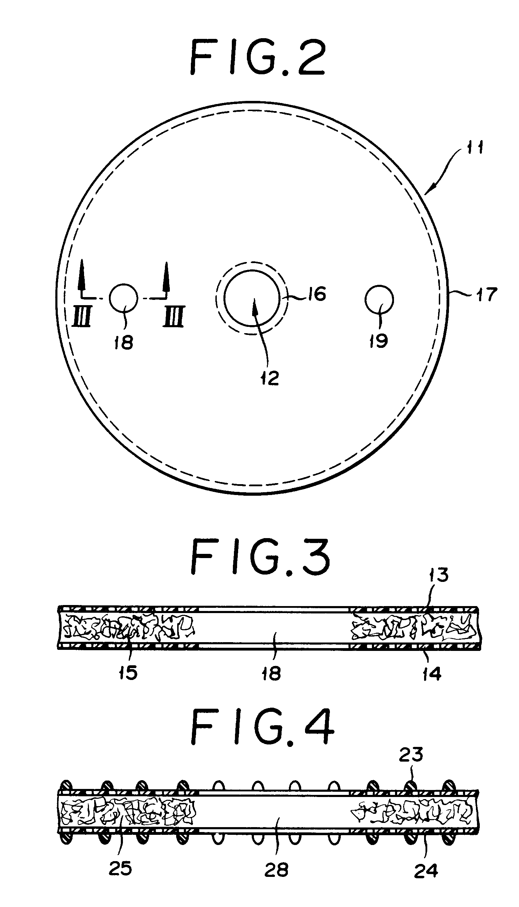

A cell-culturing substrate unit 11 is obtained by opposing at least two flat membrane type cell-culturing substrates (13) and (14) to each other with their synthetic high molecular membrane layer (not shown) sides confronting each other, interposing a flow path-forming member 15 such as non-woven fabric or net in a nipped state between the opposed substrates, and heat sealing the peripheral parts 17 and a central through hole 12 belonging in the substrates as illustrated in Fig. 2 and Fig. 3. This unit 11 generally is provided with a plurality of through holes 18 and 19 for allowing circulation of a liquid in the flow path as described specifically hereinafter. The flow path-forming member 15 is a non-woven fabric of coarse texture or a net. It fulfils its function as a space-retaining member for retaining between the substrates 13, 14 a space intended as a flow path.

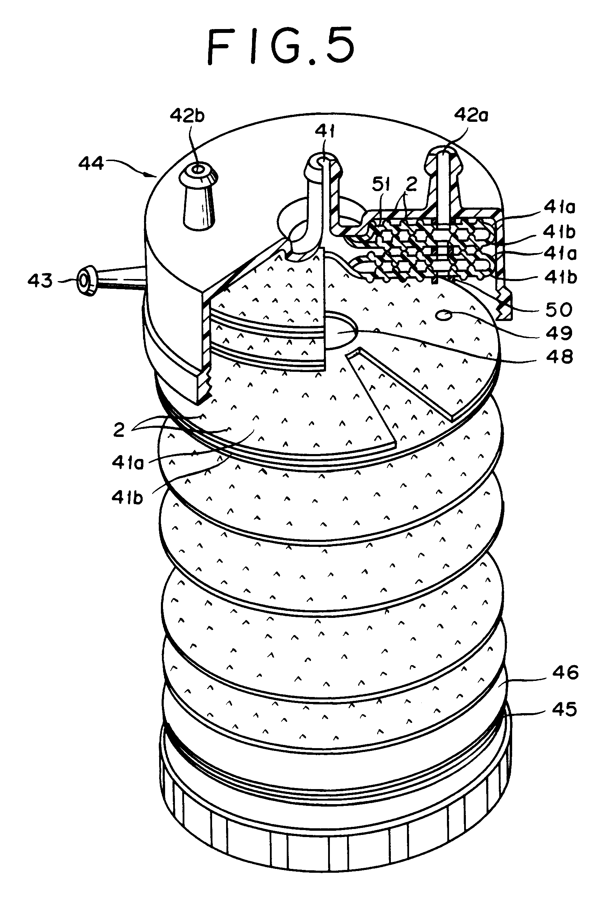

Fig. 4 is a cross section illustrating, similarly as in Fig. 3, the cell-culturing substrate unit 11 of the type having minute projections 2 formed on the obverse surface of the high molecular substrate. The reference numerals of Fig. 5 which equal those of Fig. 2 and Fig. 3 plus 10 denote the same parts.

The cell-culturing substrate unit is put to use as follows. Inside a case which comprises a cylindrical case proper 44 provided in the central part of the upper plate thereof with an inlet 41 for the useful substance-recovering liquid (hereinafter referred to as "B liquid"), in the peripheral part of the upper plate thereof with an inlet 42a for the culturing liquid (hereinafter referred to as "A liquid") and an outlet 42b for the culturing liquid, and in the lateral wall thereof with an outlet 43 for the A liquid and a bottom lid member 46 having an O-ring 45 attached to the peripheral edge thereof, a plurality of permeable membrane units 51 each formed by vertically pairing flat membrane type cell-culturing substrates 41, 41a, and 41b each provided on the opposite surfaces thereof with a plurality of minute projections 2, sealing the peripheral parts of the substrates and the peripheral parts of the central opening parts 42, and applying a sealing member 50 to the outer periphery of a filtrate permeating hole 49 are superposed as illustrated in Fig. 5. When the flat membrane type cell-culturing substrates 41 of the present invention are used as described above, the plurality of minute projections present on the surfaces of the permeable membranes keep the permeable membranes body 1 from directly contacting each other and the flow path-forming members 45 of non-woven fabric or net keep the intervening spaces between the permeable membranes proper constant at all times. Thus, the A liquid flow paths formed with the intervals of the minute projections between the adjacent cell-culturing substrate units 51 and the filtrate flow paths formed with the intervals of the flow path-forming members 45 inside the units are secured properly. Further, the A liquid flow paths formed with the intervals of the minute projections between upper surface of the uppermost unit 51 and the upper inner surface of the case and between the lower surface of the lowermost unit 51 and the bottom inner surface of the case are secured properly likewise.

Consequently, a bioreactor is formed. With this bioreactor, culture of cells or microorganisms is carried out as follows, for example. When a suspension of cells or microorganisms to be cultured (culture solution, for example) is supplied through the culture solution inlet 42a, the cells or microorganisms flow into the empty spaces intervening between the cell-culturing substrates 41a, 41b, settle on the aforementioned synthetic high molecular membrane layers (not shown), and get cultured with the culture solution (A liquid) supplied through the culture solution (A liquid) inlet 42a. The culture solution is eventually discharged through the culture solution outlet 42b.

When this operation has propagated the cells or microorganisms to a prescribed level, a useful substance-recovering solution (B liquid) is introduced through the useful substance-recovering solution inlet 41 to initiate mass transfer through the medium of the porous substrates 41a, 41b and induce passage of the useful substance produced by the cells or microorganisms into the B liquid. By allowing the B liquid to flow out through the useful substance-recovering solution outlet 43, the recovery of the useful substance is attained.

Optionally, the relation between the A liquid and the B liquid may be reversed. In this case, however, the synthetic high molecular membrane layers are preferable to be formed on the outer surfaces of the units.

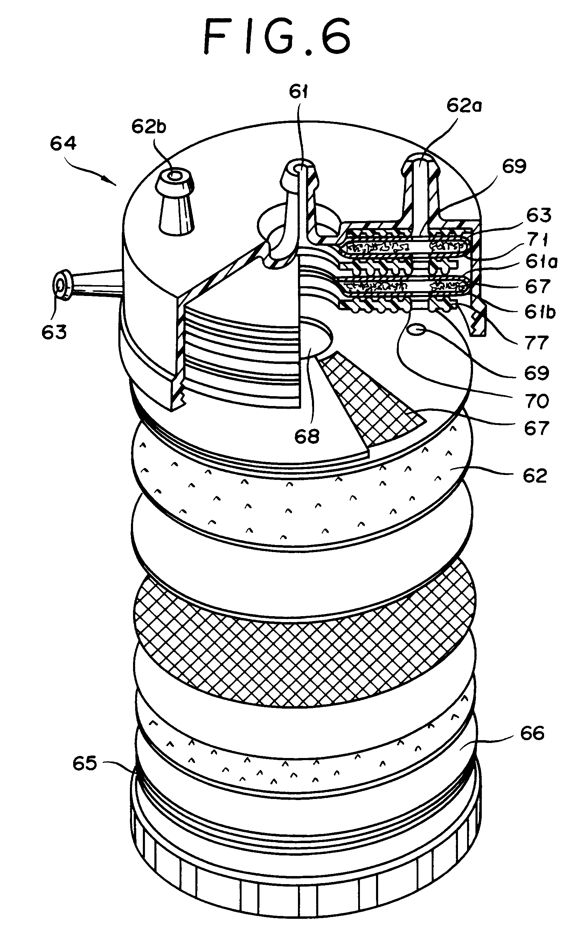

It is permissible to use cell-culturing substrate units (Fig. 2 and Fig. 3) which are devoid of minute projections as illustrated in Fig. 6. In this case, spacers 62 must be interposed between the adjacent units. The spacers 62 are generally in the form of a sheet provided on the opposite surfaces thereof with minute projections as illustrated in Fig. 1 a and Fig. 1 c. In Fig. 6, the reference numerals which equal those of Fig. 5 plus 20 denote the same parts.

In the bioreactors illustrated in Fig. 5 and Fig. 6, blood may be used as the A liquid and the culture solution as the B liquid or the blood as the B liquid and the culture solution as the A liquid. Optionally, two or more species of cells may be used for mixed culture. By culturing different species of cultures in the first compartment and the second compartment which are partitioned with a membrane, the function of cells and the life of cells can be elongated.

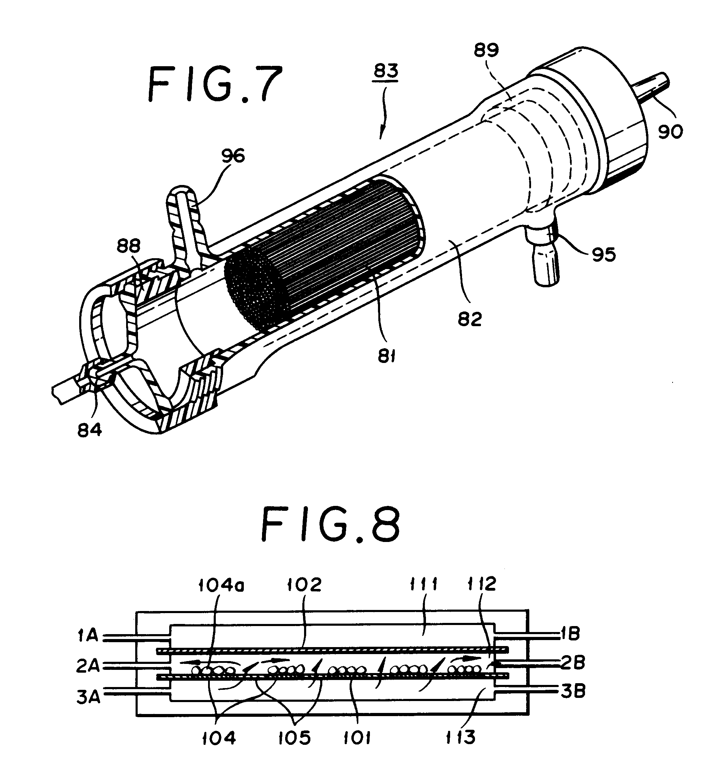

When hollow fiber membranes are used as high molecular substrates, hollow fiber substrates 81 provided on the inner surface and/or on one of the opposite surfaces with the aforementioned synthetic high molecular membrane layer are set in place in the cylindrical main body 82 of the bioreactor, with the opposite ends of the bundle of the hollow fiber substrates 81 fixed with potting agents 88, 89 to complete the bioreactor 83 as illustrated in Fig. 7. When a suspension of cells or microorganisms to be cultured is introduced under pressure, through one side port (culture solution inlet) 84 of the bioreactor 83, the cells or microorganisms flow into the hollow fibers, deposit on the synthetic high molecular membrane layers (not shown), and get cultured with the culture solution (A liquid) supplied through the culture solution (A liquid) inlet 84. The culture solution is eventually discharged through the other side port (culture solution outlet) 90.

When this operation of the bioreactor has propagated the cells or microorganisms to a prescribed level, the useful substance-recovering solution is introduced through the useful substance-recovering solution (B liquid) inlet 95 to initiate mass transfer through the medium of the hollow fiber substrates 81 and induce passage of the useful substance produced by the cells or microorganisms into the B liquid. By allowing the B liquid to be discharged through the useful substance-recovering solution outlet 96, therefore, the recovery of the useful substance mentioned above is accomplished.

The relation between the A liquid and the B liquid in the bioreactor may be reversed. In this case, the synthetic high molecular membranes are preferred to be formed on the outer surfaces of the hollow fibers.

In the bioreactors illustrated in Figs. 5 to 7, an extracorporeal circulation type culturing apparatus is formed by connecting the useful substance-recovering solution inlet to the artery or the vein of a human body and, at the same time, connecting the useful substance-recovering solution outlet to the artery or the vein of the human body and passing blood as a useful substance-recovering solution through the bioreactor.

Examples of the cells and the enzyme to be used in the present invention are as follows.

The cells which are usable herein include fibroblasts, various endothelial and epithelial cells, unstriated muscle cells, hepatic parenchymal cells, Langerhansian pancreatic cells, and various cancerated cells, for example. It is permissible to culture two or more species of cells either in a mixed state or separately in partitioned compartments.

o Hepatocytes
o Pancreatic cells
o Splenic cells
o Renal cells

The extracorporeal circulating apparatus which is operated to culture hepatocytes, therefore, can be used as an artificial liver.

The proportions and shapes of the different regions formed on the cell-culturing side surface of the cell-culturing porous membrane in the present invention are matters for free choice. Preferably, however, the surface area ratio of the region inhibiting adhesion of cells/region allowing adhesion of cells preferable to be in the range of 0.02 to 20, preferably 0.1 to 1.0 and these two regions are desired to be separated in the pattern of a lattice, stripes, or islands in sea. The thickness of the membrane is in the range of 1 to 500 $\mu$m, preferably 10 to 200 $\mu$m, the porosity of the membrane is desired to exceed 20% and preferably fall in the

EP 0 402 272 B1

range of 30 to 80%, and the pore diameter is preferred to be in the range of 0.01 to 1.0 $\mu$m, preferably 0.05 to 0.5 $\mu$m. If the thickness of the membrane exceeds 500 $\mu$m, the ability of mass transfer through the membrane is unduly degraded. If this thickness is less than 1 $\mu$m, the membrane poses problems of pinholes and insufficient strength. If the porosity is less than 20%, the membrane suffers from impairment of the ability of mass transfer and tends to entail clogging of the pores and offers hindrance to the growth of cells and the recovery of useful substance.

The cell-culturing porous membrane of the present invention is characterized by having as a main component thereof a polyolefin or a polyolefin having part or whole of hydrogen atoms thereof halogenated. The polyolefins which are usable herein include polymers (inclusive of homopolymers and copolymers) of ethylene, propylene, vinylidene chloride, ethylene chloride, vinylidene fluoride, propylene hexafluoride, tetrafluoroethylene, methyl pentene, and butadiene, for example. Since these polyolefins have no functional group in their molecular units, they have the advantage that when the molecular configurations in the region allowing adhesion of cells and the region inhibiting adhesion of cells on the membrane surface are designed and synthesized, the polyolefins are affected only sparingly by the molecules.

An untreated polyolefin which has not introduced a polar group into the surface thereof through cold plasma treatment or ozone treatment, for example, is deficient in cell adhesion and expansibility. This quality may be conversely utilized for the region inhibiting adhesion of cells. In this case, the region inhibiting adhesion of cells is preferred to have as its main component a polyolefin or a polyolefin having part or whole of the hydrogen atoms thereof halogenated.

Optionally, the region inhibiting adhesion of cells in the present invention may be given a hydrogel-like surface. The term "hydrogel-like surface" as used herein refers to what is produced by fixing a highly absorbent high molecular compound or a water-soluble high molecular compound on the surface of a membrane by insolubilization. Specifically, it is a hydrated gel texture formed in the membrane surface. The formation of this hydrated gel texture is accomplished by the method which comprises masking the part of the surface of a porous membrane destined to form the region allowing adhesion of cells and subsequently grafting an acrylamide derivative or a (meth)acrylic ester to the remaining surface of the porous membrane destined to form the region inhibiting adhesion of cells or the method which comprises coating the surface of the membrane with a polyether or a polyvinyl ether and subsequently insolubilizing the applied coating by linkage with a covalent bond or by a cross-linking insolubilizing treatment, for example. The components which are usable herein for the formation of the hydrated gel include polymers having acrylamide derivatives such as acrylamide, dimethyl acrylamide, monomethyl acrylamide, ethyl acrylamide, and isopropyl acrylamide as a component thereof and hydrated gel-forming polymers having polyethers such as polyethylene glycol, polyvinyl ethers such as polyvinyl methyl ether, and acrylic esters and methacrylic esters as a component thereof, for example.

The molecular configuration of the region allowing adhesion of cells has no particular restriction except for the requirement that it should excel compared with the region inhibiting adhesion of cells in cell adhesion and proliferation. Thus, the surface design may be carried out by any of the known methods. The method which comprises fixing cell-adhesive protein, peptide, or glycoprotein on the surface or the method which comprises introducing a weakly cationic polar group may be employed, for example. From the standpoint of practical utility, the region allowing adhesion of cells is preferred to be formed of a cell-culturing membrane having as a component thereof a compound possessing a nitrogen-containing heterocycle in the molecular unit thereof. The term "nitrogen-containing heterocycle" as used herein refers to a compound such as pyridine, pyrimidine, piperidine, pyrrolidone, indole, purine, or thiazole which has a nitrogen atom in a heterocycle. As compounds possessing a nitrogen-containing heterocycle in the molecular unit thereof, the $\alpha,\beta$-ethylenically unsaturated monomers containing a nitrogen-containing heterocycle enumerated above may be cited.

When cells are scattered on the cell-culturing porous membrane of the present invention, they are selectively caused to adhere to the cell-adhesive surface and then allowed to expand and propagate thereon. On the surface of the membrane inhibiting adhesion of cells, therefore, the clogging of the pores in the membrane with cells or with intracellular substances synthesized by the cells does not easily occur. As the result, the cell-culturing membrane of the present invention manifests an outstanding quality relative to the supply of necessary substance, removal of extraneous substance, and recovery of useful substance. When the culture of cells is carried out with the cell-culturing apparatus using the cell-culturing membrane of this invention, the function of the cells can be maintained for a long time and the produced substance can be recovered in high ratio. By having the region allowing adhesion of cells and the region inhibiting adhesion of cells formed in desired shaped separately on the surface of the membrane, the cells can be efficiently metabolized.

12

When the cell-culturing porous membrane is made of a polyolefin, it sparingly succumbs to swelling or induces enzyme degradation in an aqueous solvent and excels in dimensional stability or strength unlike the membrane made of a cellulosic material. Further, since the polyolefin itself is deficient in cell adhesion or proliferation, the part of the membrane which has escaped the surface treatment can be directly utilized as the region inhibiting adhesion of cells. Moreover, since this cell-culturing membrane is hydrophobic, it can be used on the gas-exchange side as a membrane combining the ability of cell culture with the ability of gas exchange.

When the region inhibiting adhesion of cells is furnished with a hydrogel-like surface, the adsorption of not merely the cells but also proteins and physiologically active substances to the membrane or the clogging of the pores in the membrane by such extraneous substances is curbed. Thus, the supply and recovery of useful substances can be carried out efficiently.

When the region allowing adhesion of cells has as a component thereof a compound containing a nitrogen-containing heterocycle in the molecular unit thereof, it can be handled easily as compared with the membrane made of a natural substance such as collagen. It is not degraded even under such harsh conditions as countered in the sterilization with an autoclave. When the membrane is made of a compound having an aromatic heterocycle in the molecular unit thereof, it exhibits enhanced resistance to the $\gamma$ ray and tolerates the sterilization with the $\gamma$ ray.

The cell-culturing apparatus of this invention enjoys all the merits of the cell-culturing porous membrane mentioned above.

How, the present invention will be described more specifically below with reference to working examples.

Examples 1 to 4

In a biaxial extruding device (produced by Ikegai Iron Works, Ltd. ), 100 parts by weight of polypropylenes having melt flow indexes (M.I.) of 30 and 0.3 (mixture of 100 : 40 weight ratio), 400 parts by weight of liquid paraffin as an organic filler (number average molecular weight 324), and 0.3 part by weight of 1,3,2,4-bis(p-ethyldibenzylidene) sorbitol as a crystal seed-forming agent (number average molecular weight 324) were melted, kneaded, and pelletized. The pellets are melted in the same extruding device at 150° to 200°C and extruded therefrom through a T-die having a slit width of 0.6 mm into the ambient air. The extruded sheet of the mix was led into a cooling and solidifying liquid by virtue of the rotation of a guide roller in a cooling tank located directly below the T die and the film resulting from the solidification was taken up. The rolled film was cut into pieces of a fixed length. The length of film was fixed in the directions of the longitudinal and lateral axes, immersed four times each for 10 minutes in 1,1,2-trichloro-1,2,2-trifluoroethane to expel the liquid paraffin by extraction. Then, it was heat treated in the open air at 135°C for 2 minutes, to produce a porous membrane of polypropylene containing pores 0.45 $\mu$m in average diameter and measuring 120 $\mu$m in wall thickness. The porous membrane thus obtained was exposed to cold plasma (Ar 0.1 torr) for 15 seconds. Then, a monomer such as 4-pyridyl ethylene was supplied in the gaseous state to the porous membrane and graft polymerized to the surface of the membrane at a temperature of 288K for a prescribed time. In Examples 3 and 4, two different monomers were used to effect deposition of synthetic high molecular substances formed of copolymers on the surfaces of membranes. These membranes were washed with a solvent (produced by Asahi Glass Company, Ltd. and marketed under trademark designation of "Resisol ve") for two days and dried. The dried membranes were subjected to trial culture of hepatic cells and to surface analysis.

Examples 5 and 6

The membranes obtained by the procedures of Example 2 and Example 3 were left reacting with bromoethane in methanol at 50°C for 24 hours to produce membranes having a quaternized graft chain of polyvinyl pyridine. These membranes were subjected to the same test as in Example 1.

Example 7

A solution obtained by dissolving 18 parts by weight of polyvinylidene fluoride powder (produced by Mitsubishi Petrochemical Co., Ltd. and marketed under trademark designation of "Kynar K301") in 73.8 parts by weight of acetone and 8.2 parts by weight of dimethyl formamide was cast on a polyethylene terephthalate film, then immersed in a 1,1,2-trichloro-1,2,2-trifluoroethane bath for 5 minutes, and dried to obtain a porous membrane of polyvinylidene fluoride measuring 125 $\mu$m in wall thickness and containing

EP 0 402 272 B1

pores 0.45 $\mu$m in average diameter. This porous membrane was subjected to the same surface graft polymerization as in Example 1 and then subjected to various tests.

Example 8

By following the procedure of Example 1, a porous membrane containing pores 0.6 $\mu$m in diameter and measuring 150 $\mu$m in wall thickness was obtained from a composition consisting of 100 parts by weight of polypropylene (produced by Mitsui-Toatsu Chemicals Inc. and marketed under product code of "J3H") and 60 parts by weight of ethylene-propylene random polymer (produced by Mitsui-Toatsu Chemicals Inc. and marketed under product code of "GEBG"). This membrane was exposed to the electron beam from an electron accelerator (applied pressure 1.5 MeV and electron gun current 1 mA). In an airtightly sealed container thoroughly deprived of oxygen, the membrane was placed and 4-vinyl pyridine was supplied in the gaseous state to fill up the container and subjected to surface graft polymerization, to obtain a membrane aimed at.

Control 1

The porous membrane of polypropylene prepared in Example 1 was subjected in its unmodified form to tests.

Controls 2 to 6

The porous membranes of polypropylene prepared by following the procedure of Example 1 were subjected to surface graft polymerization in the same manner as in Example 1, except that different monomers were used instead.

Control 7

A porous membrane of polyvinylidene fluoride prepared by following the procedure of Example 7 was subjected in its unmodified form to tests.

Control 8

A porous membrane of polyvinylidene fluoride prepared by following the procedure of Example 7 was subjected to cold plasma treatment under an air pressure of 0.1 torr at 100 W for 2 minutes to obtain a surface treated membrane having an oxygen/carbon ratio of 0.16. This membrane was subjected to tests.

Controls 9 to 11

Porous membranes of polyvinylidene fluoride prepared by following the procedure of Example 7 were subjected to surface graft polymerization by the procedure of Example 1 using different monomers. None of them produced a cell-culturing porous membrane aimed at by the present invention.

Control 12

A porous membrane of polyvinylidene fluoride prepared by following the procedure of Example 7 was immersed for 2 minutes in a water-methanol (1 : 1 [v/v]) solution containing 2% of vinyl alcohol-vinyl acetate copolymer (produced by Shin-etsu Chemical Industry Co., Ltd. and marketed under product code of "SMR-30L") and dried in an oven at 60°C. When the membrane was tried for ability to culture hepatic cells, no satisfactory results were obtained.

Controls 13 and 14

As commercially available culturing membranes, the membranes produced by Millipore and marketed under grademark designations of "Millicell HA" and "Millicell CM" were subjected to trial culture of hepatocytes in the same manner as in Example 1.

14

Examples 9 and 10 and Controls 15 to 18

Sample porous membranes placed in petri dishes and submerged under a suspension of a cell Eagle MEM culture medium (incorporating 10% bovine fetus blood serum) (containing 6 x $10^4$ cells/ml) were subjected at 37°C to a one week culture using cells originating in the kidneys of hamster (BHK-21) and fibroblasts originating in rats in the place of the hepatocytes.

Example 11

An extracorporeal circulation type therapeutic apparatus constructed as illustrated in Fig. 5 was produced with a flat membrane filter illustrated in Fig. 2 and Fig. 3. Fig. 3 is a magnified cross section of part of the flat-membrane filter. The flat membrane filter 11, in its plan view, assumes the shape of a doughnut possessing a through hole 12 at the center thereof. This flat membrane filter 11 was produced by opposing two cell-culturing porous membranes 11a, 11b according with the present invention, interposing a non-woven fabric 15 of coarse texture in a nipped state between the porous membranes, and airtightly joining the inner peripheries and the outer peripheries with heat seal members 16, 17. The non-woven fabric 15 of coarse texture had the function as a gap-retaining member for retaining a space serving as a fluid passage between the porous membranes 11a, 11b. It is further provided with two through holes 18, 19 for allowing circulation of a liquid through the fluid passage inside the filter.

A membrane type cell-culturing apparatus was produced by forming minute projections of UV-cured resin 80 $\mu$m in height on the outer surfaces of the flat membrane filters 11a, 11b (105 mm in outside diameter) and superposing such flat membrane filters in 10 layers within a housing of polycarbonate. In a culturing apparatus produced with cell-culturing porous membranes treated in the same manner as in Example 2, parenchymal hepatocytes separated from a male rat of one week old by the collagenase perfusion method and diluted to 5 x $10^4$ cells/mm were applied in the form of a coating to the outer sides of the filters and a culture solution (DME culture medium containing 10% bovine fetus blood serum) necessary for sustenance of cells was circulated to the interiors of the filters, to try culture of the hepatocytes. On the third and seventh days of the culture, the culturing apparatus was disassembled and the membrane surfaces were visually examined, to find that the hepatocytes were satisfactorily propagated.

Example 12

The cell-culturing substrates obtained in Examples 1 to 8 and Controls 1 to 14 were tested for adhesion, expansion, and growth of hepatocytes. To be specific, this test was conducted by separating parenchymal hepatocytes from a rat by the collagenase perfusion method proposed by Seglen, culturing the separated hepatocytes with a DME culture medium containing 10% of blood serum, examining the culture medium as to adhesion of hepatocytes on the first day of culture, as to expansion of hepatocytes on the third day of culture, and as to growth of hepatocytes on the third day of culture. The rating was made on the four-point scale (-, +, + +, and + + +) ranging from satisfactory adhesion and growth to absence of adhesion. The results are shown in Table 1.

Table 1

| | Monomer 1 | Monomer 2 | Graft ratio | $N_{15}/C_{15}$ | Adhesiveness | Expandness | Propagation |
|---|---|---|---|---|---|---|---|
| Example 1 | 1V21 | - | 3.6 | 0.07 | +++ | ++ | +++ |
| Example 2 | 4-VP | - | 6.8 | 0.09 | +++ | +++ | +++ |
| Example 3 | 4-VP | EA | 11.6 | 0.07 | +++ | +++ | +++ |
| Example 4 | 4-VP | MEA | 15.6 | 0.06 | +++ | +++ | +++ |
| Example 5 | 4-VP | - | 6.8 | 0.06 | +++ | +++ | +++ |
| Example 6 | 4-VP | EA | 11.6 | 0.06 | +++ | +++ | +++ |
| Example 7 | 4-VP | - | 7.3 | 0.10 | +++ | +++ | +++ |
| Example 8 | 2-VP | - | 5.5 | 0.07 | +++ | +++ | +++ |
| Control 1 | - | - | - | tr | - | - | - |
| Control 2 | DMAA | - | 12.6 | 0.11 | - | - | - |
| Control 3 | DMAA | EA | 20.5 | 0.05 | - | - | - |
| Control 4 | EA | - | 11.2 | tr | ++ | + | + |
| Control 5 | MAA | EA | 11.8 | tr | + | + | + |
| Control 6 | MA | | 5.7 | tr | + | + | + |
| Control 7 | - | - | - | tr | - | - | - |
| Control 8 | - | - | - | tr | ++ | ++ | + |
| Control 9 | DMAA | - | 9.1 | 0.10 | - | - | - |
| Control 10 | EA | - | 13.2 | tr | + | + | + |
| Control 11 | MAA | - | 8.7 | tr | + | + | + |
| Control 12 | - | - | - | tr | - | - | - |
| Control 13 | - | - | - | - | + | + | + |
| Control 14 | - | - | - | - | + | + | + |

16

## Example 13

The cell-culturing substrates or bioreactors of Examples 9 to 11 and Controls 15 to 18 were tested in the same manner as in Example 12. The results are shown in Table 2.

Table 2

| (Membrane of specimens) | Monomer 1 | Monomer 2 | Graft ratio | $N_{15}/C_{15}$ | Adhesiveness | Expandness | Propagation |
|---|---|---|---|---|---|---|---|
| Example 9 (Example 1) | 1V2I | - | 3.6 | 0.07 | +++ / +++ | +++ / ++ | +++ / +++ |
| Example 10 (Example 2) | 4-VP | - | 6.8 | 0.09 | +++ / +++ | +++ / ++ | +++ / +++ |
| Example 11 (Example 7) | 4-VP | - | 7.3 | 0.10 | +++ / +++ | +++ / ++ | +++ / +++ |
| Control 15 (Control 1) | - | - | - | tr | + / - | - / - | - / - |
| Control 16 (Control 2) | DMAA | - | 12.6 | 0.11 | - / - | - / - | - / - |
| Control 17 (Control 3) | DMAA | EA | 20.5 | 0.05 | + / + | - / - | - / - |
| Control 18 (Control 7) | - | - | - | tr | + / + | - / - | - / - |

In the test results on cell adhesion, expansibility, and proliferation given in Table 2, those in the upper row are data of BHK-21 and those in the lower row are data of fibroblasts.

The abbreviations used in Table 1 and Table 2 represent the following compounds:

4-VP: 4-Pyridyl ethylene MA: Methyl acrylate

2-VP: 2-Pyridyl ethylene MEA: Methoxyethyl acrylate

1V2I: 1-Vinyl-2-imidazoline MMA: Methyl methacrylate

DMAA: N,N-dimethyl acrylamide MAA: Methacrylic acid
EA: Ethyl acrylate

Example 14

Polypropylene sheets (produced by Futamura Kagaku Kogyo K.K. and marketed under product code "FOP60") 50 $\mu$m in wall thickness each having slits 3 mm in thickness punched out at intervals of 3 mm after the pattern of parallel stripes were superposed one on top of another and irradiated with cold plasma (Ar, 0.1 torr) for 15 seconds. Then, 4-pyridyl ethylene gas was supplied to the superposed polypropylene sheets and allowed to effect surface graft polymerization of the polypropylene sheets for 3 minutes at a temperature of 288 K. The superposed polypropylene sheets were separated. The membranes thus obtained were washed with solvent (produced by Asahi Glass Company, Ltd. and marketed under trademark designation of "Resinsorb") for two days and dried, to be used as samples.

A disk 29 mm in diameter was punched out of the sample. The parenchymal hepatocytes separated from a male rat 5 weeks old by the collagenase perfusion method were scattered on the disk in an amount of $5 \times 10^4$ cells and cultured for 4 days in a DE culture medium (produced by Kyokuto Seiyaku K.K.) containing 10% bovine fetus blood serum under an atmosphere consisting of 5% of carbon dioxide gas and 95% of air at 37°C. At the end of the culture, the disk coated with the cells was observed under a phase-difference microscope to find the degree of growth of cells. On the disk, regions allowing adhesion of cells and regions inhibiting adhesion of cells were formed as separated with intervals of about 3 mm after the pattern of parallel stripes. In the present example, the regions allowing adhesion of cells were parts of the surface having introduced the pyridine group as a nitrogen-containing heterocycle and the regions inhibiting adhesion of cells the parts of the surface masked by the overlying polypropylene sheet and kept from introducing the pyridine group.

Example 15

Polypropylene sheets (produced by Futamura Kagaku Kogyo K.K. and marketed under product code "FOP60") 50 $\mu$m in wall thickness each given the same surface treatment on the entire surface thereof with 4-pyridyl ethylene as in Example 1 and perforated with circular holes 5 mm in diameter separated with intervals of 10 mm after the pattern of regularly scattered waterdrops were superposed one on top of another and exposed to cold plasma (Ar, 0.1 torr) for 10 seconds. Then, N,N-dimethyl acrylamide gas was supplied to the superposed polypropylene sheets and allowed to effect surface graft polymerization of the polypropylene sheets for 3 minutes. The superposed polypropylene sheets were separated. The membranes thus obtained were washed with solvent (produced by Asahi Glass Company, Ltd. and marketed under trademark designation of "Resinsorb") for two days and dried, to be used as samples.

A disk 29 mm in diameter was punched out of the sample. The parenchymal hepatocytes separated from a male rat 5 weeks old by the collagenase perfusion method were scattered on the disk in an amount of $5 \times 10^4$ cells and cultured for 4 days in a DE culture medium (produced by Kyokuto Seiyaku K.K.) containing 10% bovine fetus blood serum under an atmosphere consisting of 5% of carbon dioxide gas and 95% of air at 37°C. At the end of the culture, the disk coated with the cells was observed under a phase-difference microscope to find the degree of growth of cells. On the disk, a circular region about 5 mm in diameter inhibiting adhesion of cells and a region surrounding the circular region and allowing adhesion of cells were separately formed after the pattern of an island in sea. In the present example, the region allowing adhesion of cells was the part of the surface having introduced a pyridine group as a nitrogen-containing heterocycle and the region inhibiting adhesion of cells was the part of the surface having a hydrogel pattern formed with the graft chain of poly(N,N-dimethyl acrylamide).

Controls 19 to 21

A polypropylene sheet (FOP 60) and a polypropylene sheet having the same hydrogel-like surface formed of the graft chain of poly(N,N-dimethyl acrylamide) as in Example 15 were used for culture of parenchymal hepatocytes. No adhesion of cells occurred on either of the polypropylene sheets.

In the case of a polypropylene sheet subjected to the same surface graft polymerization with 6-pyridyl ethylene gas at a temperature of 288 K for 3 minutes as in Example 14, while adhesion and growth of the hepatocytes were observed, no region was observed to be inhibiting adhesion of cells.

Example 16

On a porous membrane of polypropylene produced by following the procedure of Example 1, a polypropylene sheet having slits 3 mm in thickness performed at intervals of 3 mm after the pattern of parallel strips in the same manner as in Example 14 was superposed. The resultant laminate was exposed to cold plasma (Ar, 0.1 torr) for 15 seconds. Then 4-vinyl pyridine gas was supplied to the laminate and allowed to effect surface graft polymerization of the laminate at a temperature of 288 K for 3 minutes. Then, the polypropylene sheet was removed from the laminate, washed with solvent (produced by Asahi Glass Company, Ltd. and marketed under trademark designation of "Resinsorb") for 2 days and dried. The part of the surface to which 4-vinyl pyridine was grafted was a region 4 allowing adhesion of cells and the part of the surface left untreated was a region 5 inhibiting adhesion of cells. In a minimodule (having an available membrane surface about 28 cm$^2$) produced as illustrated in Fig. 8 with cell-culturing porous membranes 1 prepared as described above, high-density membrane culture of hepatocytes was tried. To be specific, the membrane of the present example was used as the first membrane 101, a gas-exchange membrane of polypropylene containing pores 0.05 $\mu$m in diameter was used as the second membrane 102, parenchymal hepatocytes 104a were sealed in the second compartment 112, a liquid culture medium (DE culture medium containing 10% bovine fetus blood serum) was passed from 3A to 3b in the third compartment 113, and a liquid culture medium was recovered via 2A and 2B. To the first compartment 111, a mixed gas consisting of 5% of carbon dioxide gas and 95% of air was passed from 1A to 1B. Even after one week following the start of the culture, the cells attained favorable growth at the sites of adhesion and, under the condition of about 20 mm Hg of TMP (pressure difference between membranes), the pores in the membrane were not clogged and allowed smooth passage of the culture medium.

Control 22

When the experiment of Example 16 was performed with a porous membrane of polypropylene having the graft chain of 4-pyridyl ethylene introduced in the entire surface thereof, On the 5th day of culture, the pores in the membrane were clogged and the recovery of the culture medium through 2B, 2A was not obtained under 20 mmHg of TMP (pressure difference between membranes).

Example 17

A polyvinylidene fluorine membrane was obtained by casting on a polyethylene terephthalate film a solution prepared by dissolving 18 parts by weight of polyvinylidene fluoride powder (produced by Mitsubishi Petro-Chemical Co., Ltd. and marketed under trademark designation of "Kynar K301") in 73.8 parts by weight of acetone and 8.2 parts by weight of dimethyl formamide. A porous membrane of polyvinylidene fluoride having a wall thickness of 125 $\mu$m and containing pores of an average diameter of 0.45 $\mu$m was obtained by immersing the aforementioned membrane in a 1,1,2-trichloro-1,2,2-trifluoroethane bath for 5 minutes and then drying the wet membrane. The same surface graft polymerization was effected on the porous membrane as in Example 3. On the porous membrane thus treated, a polypropylene sheet having slits 3 mm in thickness punched out at intervals of 3 mm after the pattern of parallel strips in the same manner as in Example 1 was superposed. The resultant laminate was exposed to cold plasma (Ar, 0.1 torr) for 15 seconds. Then, 1-vinyl-2-imidazoline gas was supplied to the laminate and allowed to effect surface graft polymerization of the laminate at a temperature of 288 K for 3 minutes. Then, the polypropylene sheet was removed from the laminate, washed with solvent (azeotropic Freon 113-methanol mixture) for two days, dried, and used for the same trial culture of hepatocytes as in Example 16.

Even after elapse of one week following the start of the culture, the cells were growing favorably at the sites of adhesion and, under the condition of about 20 mmHg of TMP (pressure difference between membranes), the pores in the membrane were not clogged and allowed smooth passage of the culture medium.

**Claims**

1. A microorganism- or cell-culturing substrate consisting of a high molecular substrate coated on at least one of its opposite surfaces by a synthetic high molecular membrane layer made up of a (co)polymer of an $\alpha,\beta$-ethylenically unsaturated monomer possessing a nitrogen-containing heterocycle in the molecular unit thereof.

2. A substrate according to claim 1, wherein said high molecular substrate is a porous membrane.

3. A substrate according to claim 1 or claim 2, wherein said nitrogen-containing heterocycle is present in said membrane layer in an amount such that the nitrogen atom/carbon atom ratio, as determined by X-ray photoelectric spectrum, is in the range of 0.01 to 0.3.

4. A substrate according to any one of claims 1 - 3, wherein said $\alpha,\beta$-ethylenically unsaturated monomer containing a nitrogen-containing heterocycle is graft polymerized in gaseous state on at least one of the opposite surfaces of said high molecular substrate.

5. A substrate according to any one of claims 2 - 4, wherein said high molecular substrate has as a main component thereof at least one member selected from the group consisting of polyolefins and polyolefins having part or whole of the hydrogen atoms thereof substituted with a halogen atom.

6. A substrate according to any one of claims 1 - 5, having minute projections spaced at substantially fixed intervals on at least one of the opposite surface of said high molecular substrate.

7. A cell-culturing substrate unit which comprises at least two cell-culturing substrates as defined in any one of claims 1 - 6 opposed to each other with their synthetic high molecular membrane layer sides confronting each other, having a seal along the matched peripheral parts of the high molecular substrates of said cell-culturing substrates, and having annular sealing members along the outer peripheries of supply mouths perforated in said substrates.

8. A bioreactor which comprises a first compartment and a second compartment for a reaction solution separated by a microorganism or cell-culturing substrate as defined in claim 2 or claim 3, and further comprising a reaction solution-exchanging mouth in said second compartment and adapted to exchange the reaction solutions for microorganisms or cells stowed in said first compartment.

9. A bioreactor according to claim 8, wherein said porous membrane type high molecular substrate is provided with through holes having an average diameter in the range of 5Å to 10$\mu$m.

10. A bioreactor according to claim 8 or claim 9, wherein said porous membrane type high molecular substrate is in the form of a hollow fiber and the outside of said microorganism- or cell-culturing substrate having said synthetic high molecular layer coated on the surface of said hollow fiber substrate constitutes itself said first compartment.

11. A bioreactor according to claim 8 or claim 9, wherein said porous membrane type high molecular substrate is in the form of a flat membrane and wherein said first compartment is formed of the interior of a cell-culturing substrate unit as defined in claim 7.

12. An extracorporeal circulation type apparatus which comprises a first compartment and a second compartment for a reaction solution separated by a microorganism- or cell-culturing substrate as defined in claim 2 or claim 3, microorganisms or cells stowed in said first compartment, and an outlet and an inlet for perfusing blood to said second compartment.

13. An apparatus according to claim 12, wherein said porous membrane type high molecular substrate is provided with through holes having an average diameter in the range of 5Å to 10$\mu$m.

14. An apparatus according to claim 12 or claim 13, wherein said porous membrane type high molecular substrate is in the form of a hollow fiber and the outside of said microorganism- or cell-culturing substrate having said synthetic high molecular layer coated on the surface of said hollow fiber substrate constitutes itself said first compartment.

15. An apparatus according to claim 12 or claim 13, wherein said porous membrane type high molecular substrate is a flat membrane and wherein said first compartment is the interior of a cell-culturing substrate unit as defined in claim 7.

**16.** A cell-culturing porous membrane provided on at least the cell-culturing side surface thereof with a region inhibiting the adhesion of cells and a region allowing the adhesion of cells, wherein said region inhibiting the adhesion of cells has a hydrogel-like surface and said region allowing the adhesion of cells is formed of a synthetic high molecular membrane layer made up of (co)polymer of an $\alpha,\beta$-ethylenically unsaturated monomer possessing a nitrogen-containing heterocycle in the molecular unit thereof.

**17.** A porous membrane according to claim 16, wherein the surface area ratio of region inhibiting adhesion of cells/region allowing adhesion of cells on said cell-culturing surface is in the range of 0.02 to 20, said two regions are separated in the form of a lattice, parallel strips, or islands in sea, the wall thickness of said membrane is in the range of 1 to 500$\mu$m, and the porosity of said membrane is not less than 20%.

**18.** A cell-culturing apparatus which comprises a porous membrane as defined in claim 16 or claim 17.

## Patentansprüche

**1.** Mikroorganismus- oder Zellkultursubstrat, bestehend aus einem hochmolekularen Substrat, das auf einer seiner sich gegenüberliegenden Oberflächen mit einer synthetischen hochmolekularen Membranschicht aus einem (Co)polymer eines $\alpha,\beta$-ethylenisch ungesättigten Monomers beschichtet ist, welches einen stickstoffhaltigen Heterocyclus in seinen Molekül besitzt.

**2.** Substrat nach Anspruch 1 oder 2, wobei das hochmolekulare Substrat eine poröse Membran ist.

**3.** Substrat nach Anspruch 1 oder 2, wobei der stickstoffhaltige Heterocyclus in der Membranschicht in einer Menge vorhanden ist, so daß das mit einem photoelektrischen Röntgenstrahlenspektrum bestimmte Stickstoff-/Kohlenstoff-Atomverhältnis im Bereich von 0,01 bis 0,3 liegt.

**4.** Substrat nach einem der Ansprüche 1 bis 3, wobei das einen stickstoffhaltigen Heterocyclus enthaltende $\alpha,\beta$-ethylenisch ungesättigte Monomer in gasförmigen Zustand auf mindestens einer der sich gegenüberliegenden Oberflächen des hochmolekularen Substrats pfropfpolymerisiert wird.

**5.** Substrat nach einem der Ansprüche 2 bis 4, wobei das hochmolekulare Substrat als Hauptbestandteil mindestens eine Substanz besitzt, ausgewählt aus der Gruppe von Polyolefinen und Polyolefinen, in denen ein Teil oder alle Wasserstoffatome durch Halogenatome ersetzt sind.

**6.** Substrat nach einem der Ansprüche 1 bis 5, das auf mindestens einer der sich gegenüberliegenden Oberflächen des hochmolekularen Substrats kleine Vorsprünge mit im wesentlichen festem Abstand voneinander aufweist.

**7.** Zellkultursubstratanlage, die mindestens zwei gegenüberliegende Zellkultursubstrate nach einem der Ansprüche 1 bis 6 aufweist, wobei sich deren synthetische hochmolekularen Membranschichten gegenüberstehen, und eine Dichtungsvorrichtung entlang den zueinanderpassenden, äußeren Teilen der hochmolekularen Substrate der Zellkultursubstrate sowie ringförmige Dichtungsteile entlang des äußeren Umfangs der in die Substrate gestanzten Versorgungsöffnungen besitzt.

**8.** Bioreaktor, der aufweist einen ersten und zweiten durch ein Mikroorganismus- oder Zellkultursubstrat nach Anspruch 2 oder 3 getrennten Teilraum für eine Reaktionslösung und ferner eine in dem zweiten Teilraum zum Austausch der Reaktionslösung befindliche Öffnung, durch die Reaktionslösungen für in dem ersten Teilraum untergebrachte Mikroorganismen oder Zellen ausgetauscht werden können.

**9.** Bioreaktor nach Anspruch 8, wobei das hochmolekulare Substrat vom Typ mit poröser Membran mit Durchtrittsöffnungen versehen ist, die einen mittleren Durchmesser im Bereich von 5 Å bis 10 $\mu$m besitzen.

**10.** Bioreaktor nach Anspruch 8 oder 9, wobei das hochmolekulare Substrat vom Typ mit poröser Membran in Form einer Hohlfaser vorliegt und die Außenseite des Mikroorganismus- oder Zellkultursubstrats mit der auf die Oberfläche des Hohlfasersubstrats aufgetragenen synthetischen hochmolekularen Schicht selbst den ersten Teilraum bildet.

**11.** Bioreaktor nach Anspruch 8 oder 9, wobei das hochmolekulare Substrat vom Typ mit poröser Membran in Form einer ebenen Membran vorliegt und der erste Teilraum aus der Innenseite einer Anlage für Zellkultursubstrat nach Anspruch 7 gebildet wird.

**12.** Vorrichtung vom extrakorporalem Umlauftyp, die aufweist einen durch ein Mikroorganismus- oder Zellkultursubstrat nach Anspruch 2 oder 3 getrennten ersten und zweiten Teilraum für eine Reaktionslösung, in dem ersten Teilraum untergebrachte Mikroorganismen oder Zellen und einen Zu- sowie Ablauf für die Perfusion von Blut in den zweiten Teilraum.

**13.** Vorrichtung nach Anspruch 12, wobei das hochmolekulare Substrat vom Typ mit poröser Membran mit Durchtrittsöffnungen versehen ist, die einen mittleren Durchmesser im Bereich von S Å bis 10 μm besitzen.

**14.** Vorrichtung nach Anspruch 12 oder 13, wobei das hochmolekulare Substrat vom Typ mit poröser Membran in Form einer Hohlfaser vorliegt und die Außenseite des Mikroorganismus- oder Zellkultursubstrats mit der auf die Oberfläche des Hohlfasersubstrats aufgetragenen synthetischen hochmolekularen Schicht selbst den ersten Teilraum bildet.

**15.** Vorrichtung nach Anspruch 12 oder 13, wobei das hochmolekulare Substrat vom Typ mit poröser Membran eine ebene Membran ist und der erste Teilraum die Innenseite einer Anlage für Zellkultursubstrat nach Anspruch 7 ist.

**16.** Poröse Membran zur Zellkultur, die wenigstens auf der Oberfläche der Zellkulturseite angebracht ist und einen Bereich zur Verhinderung der Zelladhäsion und einen Bereich zur Ermöglichung der Zelladhäsion aufweist, wobei der Bereich zur Verhinderung der Zelladhäsion eine hydrogelartige Oberfläche besitzt und der Bereich zur Ermöglichung der Zelladhäsion aus einer synthetischen hochmolekularen Membranschicht gebildet ist, die aus einem (Co)polymer eines $\alpha,\beta$-ethylenisch ungesättigten Monomers besteht, welches einen stickstoffhaltigen Heterocyclus in seinen Molekül besitzt.

**17.** Poröse Membran nach Anspruch 16, wobei das Oberflächenverhältnis des Bereichs zur Verhinderung der Zelladhäsion zum Bereich zur Ermöglichung der Zelladhäsion auf der Zellkulturoberfläche im Bereich von 0,02 bis 20 liegt, die beiden Bereiche voneinander in Form eines Gerüstes, von parallelen Streifen oder von Inseln im Meer getrennt sind, die wandstärke der Membran im Bereich von 1 bis 500 μm und die Porosität der Membran nicht unter 20 % liegt.

**18.** Zellkulturvorrichtung, die eine poröse Membran nach Anspruch 16 oder 17 aufweist.

**Revendications**

**1.** Substrat de culture de micro-organismes ou de cellules consistant en un substrat de masse moléculaire élevée recouvert sur au moins l'une de ses surfaces opposées d'une couche de membrane synthétique à haute masse moléculaire constituée par un (co)polymère d'un monomère $\alpha,\beta$-éthyléniquement insaturé possédant un hétérocycle azoté dans son unité moléculaire.

**2.** Substrat selon la revendication 1, dans lequel ledit substrat de masse moléculaire élevée est une membrane poreuse.

**3.** Substrat selon la revendication 1 ou la revendication 2, dans lequel ledit hétérocycle azoté est présent dans ladite couche de membrane en une quantité telle que le rapport atomes d'azote/atomes de carbone, tel que déterminé par le spectre photoélectrique aux rayons X, est situé dans le domaine de 0,01 à 0,3.

**4.** Substrat selon l'une quelconque des revendications 1 à 3, dans lequel ledit monomère $\alpha,\beta$-éthyléniquement insaturé contenant un hétérocycle azoté est polymérisé par greffage à l'état gazeux sur au moins l'une des surfaces opposées dudit substrat à haute masse moléculaire.

5. Substrat selon l'une quelconque des revendications 2 à 4, dans lequel ledit substrat à haute masse moléculaire comporte comme constituant principal au moins un élément choisi dans le coupe consistant en les polyoléfines et les polyoléfines dont tout ou partie des atomes d'hydrogène sont remplacés par un atome d'halogène.

6. Substrat selon l'une quelconque des revendications 1 à 5, ayant de très petites protubérances séparées par des intervalles sensiblement fixés sur au moins l'une des surfaces opposées dudit substrat à haute masse moléculaire.

7. Unité de substrat de culture de cellules qui comprend au moins deux substrats de culture de cellules tels que définis dans l'une quelconque des revendications 1 à 6 opposés l'un à l'autre et dont les côtés des couches de membranes synthétiques de masse moléculaire élevée se font face, ayant un joint d'étanchéité le long des parties périphériques appariées des substrats à haute masse moléculaire desdits substrats de culture de cellules, et ayant des éléments d'étanchéité annulaires le long des périphéries externes d'embouchures d'alimentation perforées dans lesdits substrats.

8. Bioréacteur qui comprend un premier compartiment et un second compartiment pour une solution réactionnelle séparés par un substrat de culture de micro-organismes ou de cellules tel que défini dans la revendication 2 ou la revendication 3, et comprenant en outre une embouchure d'échange de solutions réactionnelles dans ledit second compartiment et conçue pour échanger les solutions réactionnelles pour des micro-organismes ou des cellules disposés dans ledit premier compartiment.

9. Bioréacteur selon la revendication 8, dans lequel ledit substrat à haute masse moléculaire de type à membrane poreuse est muni de trous traversants ayant un diamètre moyen dans le domaine de 5 Å à 10 $\mu$m.

10. Bioréacteur selon la revendication 8 ou la revendication 9, dans lequel ledit substrat à haute masse moléculaire de type à membrane poreuse est sous forme d'une fibre creuse et l'extérieur dudit substrat de culture de micro-organismes ou de cellules ayant ladite couche synthétique à haute masse moléculaire appliquée sur la surface dudit substrat à fibre creuse constitue lui-même ledit premier compartiment.

11. Bioréacteur selon la revendication 8 ou la revendication 9, dans lequel ledit substrat à haute masse moléculaire de type à membrane poreuse est sous forme d'une membrane plaie et dans lequel ledit premier compartiment est constitué par l'intérieur d'une unité de substrat de culture de cellules telle que définie dans la revendication 7.

12. Appareil de type à circulation extracorporelle qui comprend un premier compartiment et un second compartiment pour une solution réactionnelle séparés par un substrat de culture de micro-organismes ou de cellules tel que défini dans la revendication 2 ou la revendication 3, des micro-organismes ou des cellules disposés dans ledit premier compartiment et une sortie et une entrée pour perfuser du sang dans ledit second compartiment.

13. Appareil selon la revendication 12, dans lequel ledit substrat à haute masse moléculaire de type à membrane poreuse est muni de trous traversants ayant un diamètre moyen dans le domaine de 5 Å à 10 $\mu$m.

14. Appareil selon la revendication 12 ou la revendication 13, dans lequel ledit substrat à haute masse moléculaire de type à membrane poreuse est sous forme d'une fibre creuse et l'extérieur dudit substrat de culture de micro-organismes ou de cellules ayant ladite couche synthétique à haute masse moléculaire appliquée sur la surface dudit substrat à fibre creuse constitue lui-même ledit premier compartiment.

15. Appareil selon la revendication 12 ou la revendication 13, dans lequel ledit substrat à haute masse moléculaire de type à membrane poreuse est une membrane plate et dans lequel ledit premier compartiment est l'intérieur d'une unité de substrat de culture de cellules telle que définie dans la revendication 7.

**16.** Membrane poreuse de culture de cellules munie sur au moins sa surface du côté culture de cellules d'une région inhibant l'adhésion des cellules et d'une région permettant l'adhésion des cellules, dans laquelle ladite région inhibant l'adhésion des cellules à une surface analogue à un hydrogel et ladite région permettant l'adhésion des cellules est constituée par une couche de membrane synthétique à haute masse moléculaire constitué par un (co)polymère d'un monomère $\alpha,\beta$-éthyléniquement insaturé possédant un hétérocycle azoté dans son unité moléculaire.

**17.** Membrane poreuse selon la revendication 16, dans laquelle le rapport des aires de la région inhibant l'adhésion des cellules à la région permettant l'adhésion des cellules sur ladite surface de culture de cellules est situé dans le domaine de 0,02 à 20, lesdites deux régions sont séparées sous forme d'un réseau, de bandes parallèles ou d'îles dans la mer, l'épaisseur de paroi de ladite membrane est située dans le domaine de 1 à 500 $\mu$m et la porosité de ladite membrane est supérieure ou égale à 20 %.

**18.** Appareil de culture de cellules qui comprend une membrane poreuse telle que définie dans la revendication 16 ou la revendication 17.

FIG.Ia

2

1

FIG.Ib

2

1

FIG.Ic

2

1

FIG.Id

2

1

# FIG.2

# FIG.3

# FIG.4

# FIG.5

# FIG.6

# FIG.7

# FIG.8